# FASCICULE DE BREVET EUROPEEN

(11) **EP 3 143 965 B1**
(45) Date de publication et mention de la délivrance du brevet: **26.02.2020**
(21) Numéro de dépôt: 16186875.7
(22) Date de dépôt: 01.09.2016
(51) Int. Cl.: A61F 2/46, A61F 2/30

(54) **DISPOSITIF DIT IMPACTEUR DESTINE A IMPARTIR UN CHOC A UNE TIGE**
STOSSKÖRPERVORRICHTUNG ZUM AUSFÜHREN EINES STOSSES AUF EINE STANGE
IMPACTOR DEVICE INTENDED FOR IMPARTING A SHOCK TO A ROD

(30) Priorité: 02.09.2015 FR 1501817
(43) Date de publication de la demande: 22.03.2017
(73) Titulaire: XNOV IP, 1882 Luxembourg (LU)
(72) Inventeur: BIEGUN, Jean-François, 2900 Porrentruy (CH); BIEGUN, Frédérique, 2900 Porrentruy (CH); LOEHLE, Pascal, 2900 Porrentruy (CH)
(74) Mandataire: Eidelsberg, Olivier Nathan

(56) Documents cités:
- EP-A1- 1 190 687
- EP-A1- 1 393 697
- EP-A1- 1 707 160
- EP-A1- 2 732 776

## Description

La présente invention se rapporte à un dispositif dit impacteur destiné à impartir un choc à une tige, la tige ayant pour fonction de transmettre ce choc à un insert, par exemple un insert en céramique, pour l'insérer dans un élément de réception, par exemple une cupule destinée à être positionnée dans la cavité cotyloïdienne d'une hanche.

Aujourd'hui, dans les dispositifs connus, pour insérer un insert dans une cupule destinée à être placée ensuite dans la cavité cotyloïdienne d'une hanche, on saisit l'insert par l'intermédiaire d'un dispositif de prise comportant une tige, on positionne l'insert pris par le dispositif de prise au-dessus de la cavité ouverte de la cupule et, à l'aide d'un dispositif impacteur sous la forme d'un marteau, le chirurgien applique un choc à la tige pour faire pénétrer l'insert dans la cupule.

Ces dispositifs et procédés de l'art antérieur sont peu précis et, en particulier, il survient souvent que la force appliquée par le choc ne soit ni de la bonne valeur et/ou ni parfaitement centrée, ce qui entraîne une insertion désaxée et/ou incomplète de l'insert dans la cupule, obligeant le chirurgien la plupart du temps à recommencer avec un nouvel insert et une nouvelle cupule, car l'insert mal inséré dans la cupule ne peut plus en être retiré. Il peut, en outre, arriver que le choc en raison de la mauvaise insertion soit trop fort, notamment compte tenu du mauvais axe d'orientation et entraîne une fêlure dans l'insert et/ou la cupule préjudiciable à la durée de vie de la prothèse destinée à être insérée dans la cotyle.
Il est connu de EP 1 393 697 un dispositif impacteur qui réalise un impact sur un élément qui reste toujours dans la chambre du marteau.
La présente invention vise à surmonter les inconvénients de l'art antérieur en proposant un dispositif formant impacteur qui permet d'assurer au chirurgien réalisant l'impaction d'un insert dans une cupule que, quelle que soit sa forme du jour, il applique la bonne valeur de choc, et suivant la bonne direction d'application, de sorte que l'insert est inséré chaque fois convenablement dans la cupule, diminuant ainsi drastiquement le nombre de fois où le chirurgien doit s'y reprendre à plusieurs fois ou utiliser un nouveau couple insert-cupule en remplacement du couple insert-cupule détérioré par une première tentative d'insertion défectueuse.
Suivant l'invention, un dispositif impacteur est destiné à impartir un choc à une tige, notamment indépendante du dispositif, la tige étant elle-même couplée à un élément formant insert destiné à être inséré dans un élément de réception, notamment un insert en céramique dans une cupule d'une cavité cotyloïdienne de hanche.

L'invention consiste en un ensemble comportant un dispositif impacteur et une tige, tel que défini à la revendication 1.

En prévoyant ainsi de n'impartir le choc que lorsque le contact tige-dispositif impacteur est parfaitement réalisé, notamment avec une pression suffisante du dispositif sur la tige, on s'assure que la transmission du choc s'effectue sans déperdition d'énergie et suivant le bon axe, l'extrémité de la tige étant assurée de se trouver exactement là où elle doit être pour que les moyens d'application de choc lui administrent le choc d'une puissance telle que prévue et suivant l'exacte bonne direction, diminuant ainsi drastiquement les applications avec un choc d'une valeur erronée et/ou désaxé, notamment plus faible que la valeur théorique censée être appliquée. On obtient ainsi un système particulièrement fiable pour assurer au chirurgien une frappe aussi précise que possible de la tige et donc une insertion suivant le bon axe et avec la bonne force de l'insert dans la cupule. D'autre part, le système suivant l'invention est particulièrement simple d'utilisation et en particulier le nettoyage du système est facilité par le fait que la tige est indépendante du corps du dispositif.
Suivant un mode de réalisation préféré de l'invention, les moyens de mise en précontrainte sont agencés de manière à bloquer l'action des moyens d'application de choc sur la tige tant que l'utilisateur n'applique pas le dispositif impacteur contre la tige avec la pression minimale déterminée à l'avance, l'agencement étant tel que lorsque la pression minimale déterminée à l'avance est appliquée les moyens d'application de choc peuvent être actionnés par les moyens de déclenchement.

Selon l'invention, les moyens de mise en précontrainte comportent une glissière montée coulissante par rapport à l'élément formant canon. La glissière peut être montée sous la contrainte d'un ressort tendant à maintenir l'extrémité distale de la glissière à distance de l'ouverture distale du canon.
Suivant un mode de réalisation avantageux, la glissière coopère avec les moyens de déclenchement de sorte qu'elle bloque toute action des moyens de déclenchement, et lorsque le canon est pressé contre la glissière, celle-ci libère les moyens de déclenchement.
De préférence, les moyens de déclenchement comportent une gâche commandée par une détente, la gâche butant contre la glissière dans la position dans laquelle il n'est pas exercée de pression du dispositif contre la tige avec ladite pression minimale déterminée à l'avance et étant libéré par la glissière lorsque au moins la pression minimale déterminée à l'avance est exercée.
Suivant un mode de réalisation perfectionné, il est prévu des moyens d'encliquetage, notamment sous la forme d'une bille d'encliquetage reçue dans un alésage perpendiculaire à l'axe du canon et débouchant dans le canal recevant la tige, et d'une cuvette formé dans la tige, dans laquelle la bille pénètre, destinés à assurer une limitation partielle du déplacement relatif de la tige entre deux positions d'extrémité proximale et distale correspondant aux extrémités distale et proximale respectives de la cuvette, dans la position proximale la tige étant exactement à l'endroit où il convient qu'elle soit pour être impacté tel que prévu et dans la position distale, la tige étant en retrait du canal de grand diamètre.

Suivant l'invention, il est prévu des moyens formant butée issue de la tige destinés à buter contre le bord de l'ouverture distale de la glissière.

A titre d'exemple, on décrit maintenant un mode de réalisation préféré de l'invention en se reportant aux dessins dans lesquels :
- la figure 1: est une vue en perspective d'un mode de réalisation d'un dispositif impacteur suivant l'invention ;
- la figure 2: est une vue de côté sous une forme schématique du dispositif de la figure 1, dans laquelle un tronçon d'extrémité proximale de la tige destinée à recevoir l'impact a été insérée par l'extrémité distale ouverte du dispositif impacteur ;
- la figure 3A: est une vue en coupe longitudinale d'une partie du dispositif de la figure 2 dans une position de repos, dans laquelle l'action de la détente et de la gâchette est bloquée par la glissière ;
- la figure 3B: est une vue sensiblement identique à celle de la figure 3A dans une position dite de frappe, dans laquelle la gâchette n'est plus bloquée par la glissière, de sorte le marteau peut être actionnée par appui sur la détente ;
- la figure 4A: est une vue de dessous à l'envers d'une partie du dispositif de la figure 2, au niveau de la coopération entre la glissière et la gâche, dans une position de blocage ou de sécurité du dispositif ;
- la figure 4B: est une vue identique à la figure 4A, mais dans une position permettant la frappe de la tige par le marteau, la gâche n'étant plus bloquée par la glissière ;
- la figure 5A: est une vue en perspective détaillée d'une partie de la figure 3A ;
- la figure 5B: est une vue en perspective de la partie d'extrémité proximale de la tige insérée dans le dispositif impacteur à la figure 5A;
- la figure 6: est une vue en coupe longitudinale d'une partie de la figure 2 au niveau de la crosse, dans la position bloquée de la gâchette ;
- la figure 7: est une vue en coupe transversale suivant la ligne A-A de la figure 6.

Aux figures, il est représenté de manière générale aussi bien un dispositif impacteur suivant un mode de réalisation de l'invention qu'un assemblage comportant ce dispositif impacteur et une tige insérée dans le dispositif impacteur.

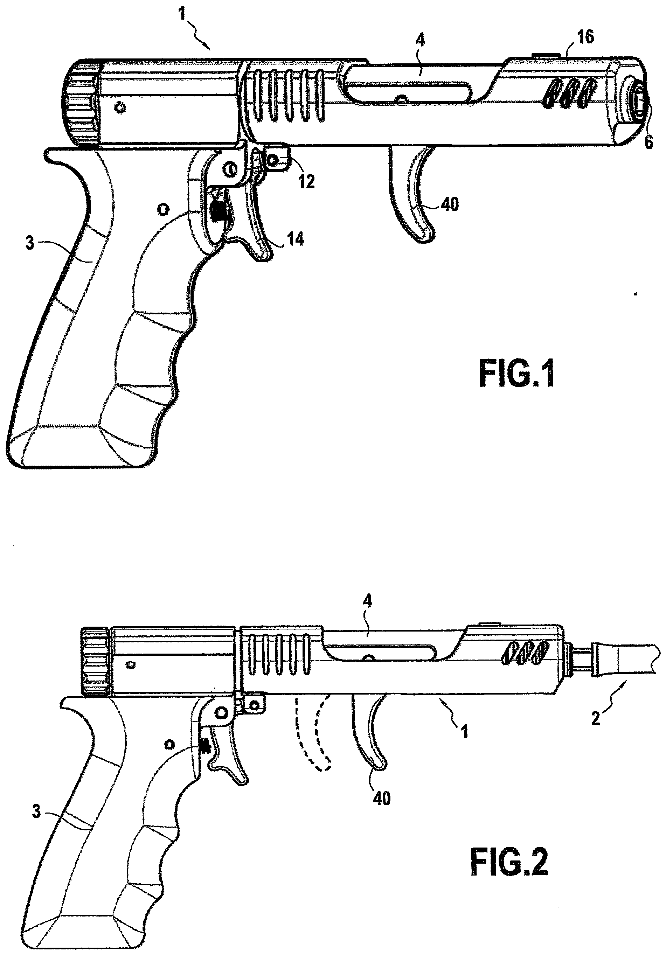

A la figure 1, il est représenté le dispositif impacteur et une tige suivant l'invention. Le dispositif 1 impacteur a la forme générale d'un pistolet. Une tige 2 est insérée en partie dans le canon du pistolet 1.

Le dispositif impacteur en forme de pistolet comporte un corps principal comportant une poignée 3 en forme de crosse de pistolet et un élément 4 sensiblement tubulaire formant canon s'étendant à partir de la crosse. L'élément 4 tubulaire formant canon définit en son sein un canal 5 intérieur débouchant à une ouverture 6 d'extrémité distale de l'élément en forme de pistolet, ouverture 6 par laquelle pénètre la tige 2.

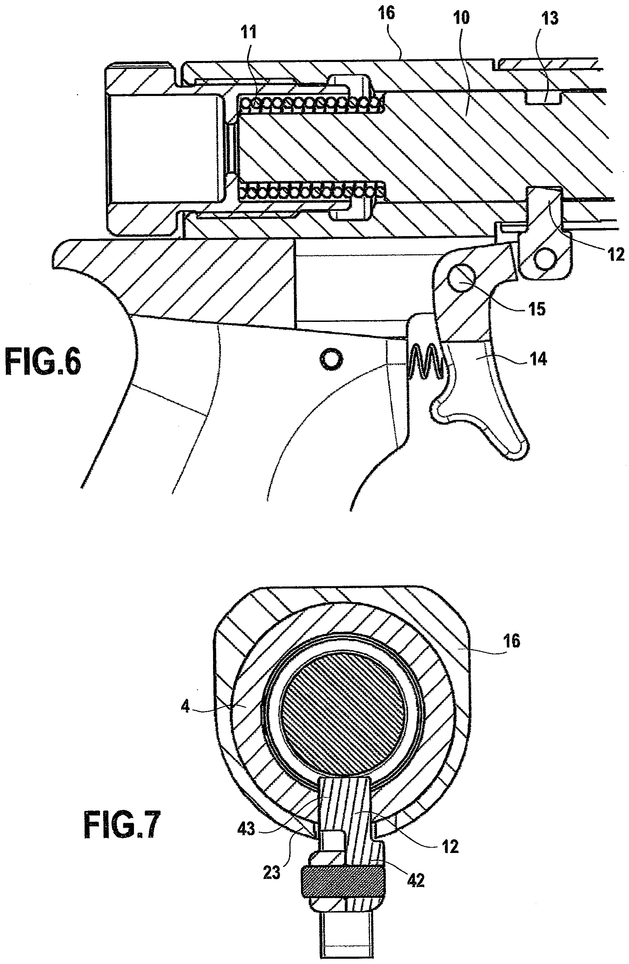

Le canal 5 intérieur comporte un tronçon 7 proximal de grande section transversale et un tronçon 8 distal de plus petite section à la suite du tronçon 7 de grande section, les deux tronçons 7, 8 étant séparés par un épaulement 9. La petite section du tronçon 8 est sensiblement égale à celle de la partie de la tige 2 destinée à être insérée dans le pistolet et est plus petite que la section du tronçon 7. Dans le tronçon 7 est disposé un marteau 10 en forme de poussoir cylindrique de section sensiblement identique à celle du tronçon de grande section qui est monté sous la sollicitation d'un ressort 11. Le marteau 10 est maintenu contre le ressort 11 à l'état comprimé par un ergot formant gâche 12 qui pénètre dans une rainure 13 annulaire du marteau. L'ergot formant la gâche 12 comporte une partie formant tige 42 s'étendant perpendiculairement à l'axe du canon et une partie formant tête 43 faisant saillie latéralement de la tige 42 pour définir une zone de crochetage, dont la fonction sera définie plus loin dans la description en liaison avec une glissière 16. Une détente 14 est montée pivotante par rapport à la crosse et est articulée à la tige 42 de la gâche 12, de sorte que lorsque l'utilisateur tient le dispositif par la crosse, il peut appuyer avec un doigt, notamment l'index, sur la détente 14 pour la faire pivoter dans le sens des aiguilles d'une montre, à la figure 6, par rapport à son axe 15 de rotation (axe perpendiculaire au sens de la figure), cela ayant pour effet de faire se déplacer vers le bas la gâchette 12 pour la faire sortir de la rainure 13 et libérer le marteau 10 vers la tige (vers la droite à la figure 6) sous l'effet de la poussée du ressort 11 libéré. Le marteau 10 peut alors venir frapper l'extrémité proximale de la tige 2 insérée dans le canal 8 et ayant pénétré en partie dans le canal 7 de grande section.

Le dispositif pistolet comporte un élément formant glissière 16 de forme tubulaire qui entoure, dans une grande mesure, le canon 4. La glissière 16 peut coulisser par rapport au canon 4. A son extrémité distale, la glissière 16 comporte une ouverture 17 distale en vis-à-vis de l'ouverture 6 distale du canon 4 pour permettre le passage de la tige 2 au sein du canal 5 défini dans l'élément 4 tubulaire formant le canon.

Un ressort 20 hélicoïdal de glissière est monté à l'intérieur de la glissière 16, entre la glissière 16 et le canon 4. Le ressort porte d'une part contre la face 19 intérieure latérale distale de la glissière et contre une butée annulaire 21 formée à la face extérieure du canon 4, à distance de l'ouverture 6 distale. Le ressort est agencé de manière à maintenir élastiquement à distance la face 19 intérieure latérale distale de la glissière et le bord distal de l'ouverture 6 distale du canon 4.

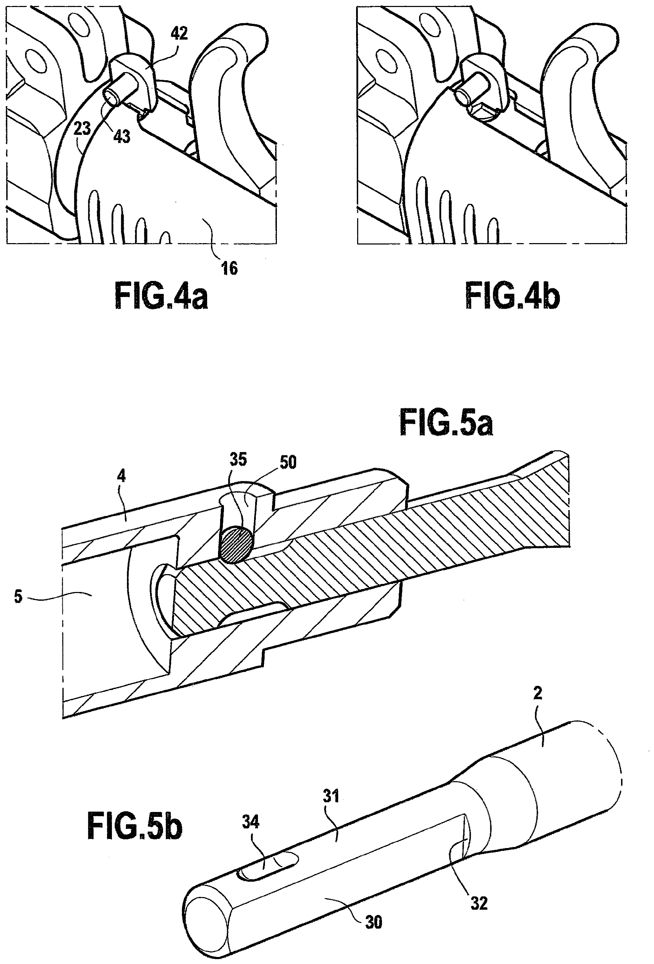

La tige 2, sensiblement cylindrique, est représentée en détail, en ce qui concerne un tronçon d'extrémité proximale, à la figure 5B. Ce tronçon proximal comporte deux méplats 30 et 31 gauche et droit opposés qui ont une extension dans la direction longitudinale de la tige qui est supérieure à l'extension en longueur du tronçon 8 de petit diamètre du canal 5. La forme de la section transversale du tronçon 8 du canal 5 correspond à celle du tronçon à méplats de la tige, de sorte que lorsque la partie de la tige comportant les méplats est introduite dans le canal 5, cela ne peut se faire que pour une orientation relative donnée de la tige et du canon, les méplats empêchant ensuite toute rotation de la tige par rapport au canon.

La tige comporte deux butées 32 respectives à la fin des méplats 30 et 31 gauche et droit, de sorte que la tige ne peut pas être introduite dans le canal 5 plus avant lorsque ces butées 32 viennent buter contre le bord extérieur de l'ouverture distale de la glissière 16.

La tige comporte aussi deux cuvettes 34 supérieure et inférieure formées dans la face latérale de la tige 2 s'étendant entre les deux méplats.

Ces deux cuvettes peuvent chacune recevoir une bille 35 poussoir d'encliquetage, montée sur ressort de manière à faire saillie en partie à l'intérieur du tronçon 8 de canal. La bille 35 poussoir est montée dans un alésage 50 perpendiculaire à l'axe du canon en étant sous une sollicitation élastique, par exemple d'un ressort (non visible aux figures) qui tend à la maintenir en partie à l'intérieur du tronçon 8 tout en pouvant cependant, sous une action de poussée de la tige lors de son introduction dans le canal, être repoussée à l'intérieur de l'alésage 50, pour permettre le passage de la tige, la bille poussoir revenant, après passage de la partie proximale sans cuvette de la tige, dans une des deux cuvettes, par exemple la cuvette supérieure.

Les dimensions relatives de la tige, notamment des méplats 30 et 31, des butées 32, des cuvettes 34, de la bille 35, du tronçon 8, de la butée 21 annulaire, du canon et de la glissière sont choisies de sorte que d'une part lorsque les butées axiales 32 de la tige bute contre le bord extérieur de l'ouverture distale de la glissière 16 et que le ressort entre la glissière et le canon est au repos, l'extrémité proximale de la tige ne dépasse pas du tronçon 8 et en particulier ne pénètre pas dans le tronçon 7 de grande section et la bille 35 est reçue en partie dans la cuvette 34 supérieure et bute contre le bord proximal de la cuvette 34 supérieure et d'autre part lorsque le ressort est comprimé par une action de l'utilisateur pressant le dispositif, notamment la glissière, contre la tige de sorte qu'il y ait contact entre la face intérieure distale de la glissière et le bord de l'ouverture distale du canon, la bille poussoir bute contre le bord distal de la cuvette et la tige a pénétré dans le canal 7 de grande section, exactement dans la bonne position relative par rapport au marteau pour que soit délivré le choc tel qu'il doit l'être.

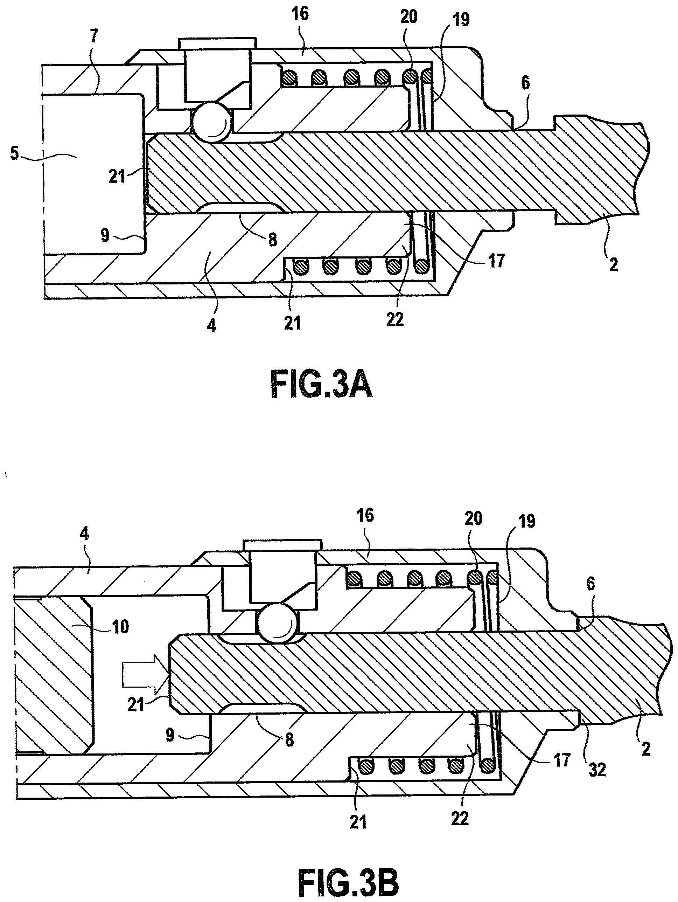

Du côté proximal (voir notamment les figures 4a et 4b) la glissière 16 sensiblement tubulaire est ouverte. Dans une position dite de sécurité du dispositif, et notamment une position de repos du ressort 20 de glissière (figures 3a et 4a), le bord latéral proximal 23 de la glissière est crocheté par la tête 43 de la gâche 12, ce qui annihile tout mouvement de celle-ci, notamment vers le bas (vers le haut à la figure 3a).

Lorsque l'utilisateur, ayant pris la crosse dans une main, presse le dispositif contre la tige, cela a pour effet de faire glisser le canon par rapport à la glissière contre la compression du ressort 20, jusqu'à ce que l'extrémité avant du canon vienne contre la paroi latérale intérieure de la glissière, la glissière se décalant alors par rapport à la tête de la gâche (figure 4b) et libérant cette dernière qui vient en correspondance avec une fente formée dans la partie inférieure de la glissière, la fente ayant une dimension approprié pour permettre sa traversée par la tête de la gâche. L'utilisateur peut alors activer le marteau pour frapper la tige qui est dans la bonne position, notamment l'extrémité proximale de la tige.

L'action relative de la bille poussoir et de la cuvette réalise une fonction d'encliquetage qui permet à l'utilisateur d'être informé, lorsqu'il presse le dispositif contre la tige, de la position dans laquelle il se trouve, notamment du moment où la gâchette a été libérée.

Une patte 40 est solidaire de la glissière 16 et aide l'utilisateur pour faire coulisser la glissière, notamment contre la tension du ressort 20. A la figure 2, le dispositif est représenté dans la position désarmée ou de sécurité. En pointillés, il est représenté la position dans laquelle se trouve la patte 40 dans la position armée ou débloquée. En revanche, la glissière, à la figure 2, n'est représentée que dans la position de sécurité.

## Revendications

1. Ensemble comportant un dispositif (1) impacteur et une tige, le dispositif étant destiné à impartir un choc à la tige, la tige étant elle-même couplée à un élément formant insert destiné à être inséré dans un élément de réception, notamment un insert en céramique dans une cupule d'une cavité cotyloïdienne de hanche ;
le dispositif (1) impacteur comportant :
- un élément (4) formant canon définissant un canal (5) ou chambre intérieure ayant une ouverture par laquelle est insérée au moins une partie de la tige, notamment un tronçon d'extrémité proximale de la tige ;
- des moyens (10, 11) d'application de choc destinés à appliquer à la partie de la tige introduite dans la chambre un choc de puissance déterminée à l'avance ; et
- des moyens (12, 13, 14, 42, 43) de déclenchement des moyens d'application de choc,
**caractérisé en ce qu'**il est prévu des moyens de mise en précontrainte qui permettent à l'utilisateur de presser le dispositif contre la tige avant de déclencher les moyens de déclenchement et donc l'action du marteau sur la tige, l'agencement étant tel que dans au moins une première position dite d'application de choc, le tronçon d'extrémité proximale de la tige se trouve au moins en partie à l'intérieur du canal et dans une deuxième position, après l'application du choc, le tronçon d'extrémité proximale de la tige se trouve à l'extérieur du canal (5),
les moyens de mise en précontrainte comportant une glissière (16) montée coulissante par rapport à l'élément formant canon, et
il est prévu des moyens formant butée (32) issue de la tige destinés à buter contre le bord de l'ouverture distale de la glissière.

2. Ensemble suivant la revendication 1, **caractérisé en ce que** les moyens de mise en précontrainte sont agencés de manière à bloquer l'action des moyens d'application de choc sur la tige tant que l'utilisateur n'applique pas le dispositif impacteur contre la tige avec la pression minimale déterminée à l'avance, l'agencement étant tel que lorsque la pression minimale déterminée à l'avance est appliquée les moyens d'application de choc peuvent être actionnés par les moyens de déclenchement.

3. Ensemble suivant la revendication 1 ou 2, **caractérisé en ce que** la glissière est montée sous la contrainte d'un ressort (20) tendant à maintenir l'extrémité distale de la glissière à distance de l'ouverture distale du canon.

4. Ensemble suivant la revendication 3, **caractérisé en ce que** la glissière (16) coopère avec les moyens de déclenchement de sorte qu'elle bloque toute action des moyens de déclenchement, et lorsque le canon est pressé contre la glissière, celle-ci libère les moyens de déclenchement.

5. Ensemble suivant la revendication 3 ou 4, **caractérisé en ce que** les moyens de déclenchement comportent une gâche (12) commandée par une détente (14), la gâche butant contre la glissière (16) dans la position dans laquelle il n'est pas exercée de pression du dispositif contre la tige avec ladite pression minimale déterminée à l'avance et étant libéré par la glissière lorsque au moins la pression minimale déterminée à l'avance est exercée.

6. Ensemble suivant la revendication 1, **caractérisé en ce qu'**il est prévu des moyens d'encliquetage destinés à assurer une limitation partielle du déplacement relatif de la tige entre deux positions d'extrémité proximale et distale, dans la position proximale la tige étant exactement à l'endroit où il convient qu'elle soit pour être impacté tel que prévu et dans la position distale, la tige étant en retrait d'un canal de grand diamètre.

7. Ensemble suivant la revendication 6, **caractérisé en ce que** les moyens d'encliquetage sont sous la forme d'une bille (35) d'encliquetage reçue dans un alésage (50), notamment perpendiculaire à l'axe du canon, et débouchant dans le canal recevant la tige, et d'une cuvette (34) formé dans la tige, dans laquelle la bille pénètre, les deux positions d'extrémité proximale et distale correspondant aux extrémités distale et proximale respectives de la cuvette.

8. Assemblage comportant un ensemble suivant l'une des revendications 1 à 7, un insert couplé à la tige et une cupule, dans laquelle l'insert est destiné à être impacté.

## Patentansprüche

1. Gesamtheit, welche eine Stoßkörpervorrichtung (1) und eine Stange umfasst, wobei die Vorrichtung dazu bestimmt ist, einen Stoß auf die Stange auszuführen, wobei die Stange ihrerseits mit einem Element gekoppelt ist, das einen Einsatz bildet, der dazu bestimmt ist, in ein Aufnahmeelement eingesetzt zu werden, insbesondere einen Einsatz aus Keramik in einer Hüftgelenkspfanne;
wobei die Stoßkörpervorrichtung (1) umfasst:
- ein einen Lauf bildendes Element (4), das einen Kanal (5) oder eine innere Kammer mit einer Öffnung definiert, durch welche wenigstens ein Teil der Stange, insbesondere ein proximaler Endabschnitt der Stange, eingesetzt wird;
- Mittel (10, 11), zur Stoßausführung, die dazu bestimmt sind, auf den in die Kammer eingeführten Teil der Stange einen Stoß mit einer vorbestimmten Stärke auszuführen; und
- Mittel (12, 13, 14, 42, 43) zur Auslösung der Mittel zur Stoßausführung,
**dadurch gekennzeichnet, dass** Mittel zum Vorspannen vorgesehen sind, die einem Benutzer ermöglichen, die Vorrichtung gegen die Stange zu drücken, bevor die Mittel zur Auslösung und somit die Einwirkung des Hammers auf die Stange ausgelöst werden, wobei die Anordnung so ist, dass in wenigstens einer ersten Position, Stoßausführungsposition genannt, der proximale Endabschnitt der Stange sich wenigstens teilweise im Inneren des Kanals befindet und in einer zweiten Position, nach der Ausführung des Stoßes, der proximale Endabschnitt der Stange sich außerhalb des Kanals (5) befindet, wobei die Mittel zum Vorspannen eine Gleitführung (16) umfassen, die in Bezug auf das einen Lauf bildende Element gleitend gelagert ist, und Mittel vorgesehen sind, die einen an die Stange angeformten Anschlag (32) bilden und dazu bestimmt sind, am Rand der distalen Öffnung der Gleitführung zur Anlage zu kommen.

2. Gesamtheit nach Anspruch 1, **dadurch gekennzeichnet, dass** die Mittel zum Vorspannen so ausgebildet sind, dass sie die Einwirkung der Mittel zur Stoßausführung auf die Stange blockieren, solange der Benutzer nicht die Stoßkörpervorrichtung mit dem vorbestimmten minimalen Druck gegen die Stange drückt, wobei die Anordnung so ist, dass, wenn der vorbestimmte minimale Druck ausgeübt wird, die Mittel zur Stoßausführung von den Mitteln zur Auslösung betätigt werden können.

3. Gesamtheit nach Anspruch 1 oder 2, **dadurch gekennzeichnet, dass** die Gleitführung unter der Vorspannung einer Feder (20) angebracht ist, die bestrebt ist, das distale Ende der Gleitführung in einem Abstand von der distalen Öffnung des Laufes zu halten.

4. Gesamtheit nach Anspruch 3, **dadurch gekennzeichnet, dass** die Gleitführung (16) mit den Mitteln zur Auslösung derart zusammenwirkt, dass sie jede Wirkung der Mittel zur Auslösung blockiert, und dass, wenn der Lauf gegen die Gleitführung gedrückt wird, diese die Mittel zur Auslösung freigibt.

5. Gesamtheit nach Anspruch 3 oder 4, **dadurch gekennzeichnet, dass** die Mittel zur Auslösung ein Schließblech (12) umfassen, das von einem Abzug (14) gesteuert wird, wobei das Schließblech in der Position, in welcher kein Druck der Vorrichtung gegen die Stange mit dem vorbestimmten minimalen Druck ausgeübt wird, an der Gleitführung (16) anliegt und von der Gleitführung freigegeben wird, wenn wenigstens der vorbestimmte minimale Druck ausgeübt wird.

6. Gesamtheit nach Anspruch 1, **dadurch gekennzeichnet, dass** Rastmittel vorgesehen sind, die dazu bestimmt sind, eine teilweise Begrenzung der relativen Verlagerung der Stange zwischen zwei Endpositionen, einer proximalen und einer distalen, sicherzustellen, wobei sich die Stange in der proximalen Position genau an der Stelle befindet, wo sie sein muss, um so wie vorgesehen gestoßen zu werden, und wobei die Stange in der distalen Position in einen Kanal mit großem Durchmesser zurückgezogen ist.

7. Gesamtheit nach Anspruch 6, **dadurch gekennzeichnet, dass** die Rastmittel die Form einer Rastkugel (35), die in einer Bohrung (50) aufgenommen wird, die insbesondere senkrecht zur Achse des Laufes ist und in den die Stange aufnehmenden Kanal mündet, und einer in der Stange ausgebildeten Schale (34), in welche die Kugel eindringt, aufweisen, wobei die zwei Endpositionen, die proximale und die distale, dem distalen bzw. proximalen Ende der Schale entsprechen.

8. Anordnung, welche eine Gesamtheit nach einem der Ansprüche 1 bis 7, einen Einsatz, der mit der Stange gekoppelt ist, und eine Pfanne, in welche der Einsatz gestoßen zu werden bestimmt ist, umfasst.

## Claims

1. Unit comprising an impaction device (1) and a stem, said device being intended to impart a blow to the stem, the stem itself being coupled to an element forming an insert intended to be inserted into a receiving element, in particular a ceramic insert in a cup of a cotyloid cavity of a hip;
the impaction device (1) comprising:
- an element (4) forming a barrel defining a channel (5) or an internal chamber that has an opening via which at least part of the stem, in particular a proximal end segment of the stem, is inserted;
- means (10, 11) of applying a blow intended to apply to the part of the stem introduced into the chamber a pre-determined blow force; and
- means (12, 13, 14, 42, 43) for triggering the blow application means,
**characterized in that** pre-stressing means are provided which enable the user to press the device against the stem before triggering the triggering means and so the action of the hammer on the stem, the arrangement being such that in at least a first so-called blow application position, the proximal end segment of the stem is located at least partially within the channel, and in a second position, after application of the blow, the proximal end segment of the stem is located outside of the channel (5),
wherein the pre-stressing means comprise a runner (16) that is fitted so as to slide relative to the element forming the barrel, and
means are provided that form a stop (32) originating from the stem intended to strike against the edge of the distal opening of the runner.

2. The unit according to claim 1, **characterized in that** the pre-stressing means are arranged such as to block the action of the means of applying a blow to the stem if the user is not applying the impaction device against the stem with the pre-determined minimum pressure, the arrangement being such that when the pre-determined minimum pressure is applied, the means for applying the blow can be actuated by the triggering means.

3. The unit according to claim 1 or 2, **characterized in that** the runner being fitted stressed by a spring (20) that keeps the distal end of the runner away from the distal opening of the barrel.

4. The unit according to claim 3, **characterized in that** the runner (16) cooperates with the triggering means such that it blocks any action of the triggering means, and when the barrel is pressed against the runner, the latter releases the triggering means.

5. The unit according to claim 3 or 4, **characterized in that** the triggering means comprise a striker (12) controlled by a trigger (14), the striker hitting the runner (16) in the position in which the device is not exerting any pressure upon the stem with said pre-determined minimum pressure and being released by the runner when at least the pre-determined minimum pressure is being exerted.

6. The unit according to claim 1, **characterized in that** locking means are provided intended to provide partial limitation of the relative movement of the stem between two proximal and distal end positions, in the proximal position the stem being in exactly the place where it should be in order to be impacted as intended, and in the distal position the stem being withdrawn from the channel with a large diameter.

7. The unit according to claim 6, **characterized in that** the locking means are in the form of a locking ball (35) received in a borehole (50), in particular perpendicular to the axis of the barrel, and opening out into the channel that receives the stem, and of a depression (34) formed in the stem, into which the ball penetrates, the two proximal and distal end positions corresponding to the respective distal and proximal ends of the depression.

8. An assembly comprising a unit according to any one of claims 1 to 7, an insert coupled to the stem and a cup in which the insert is intended to be impacted.
